# EUROPÄISCHE PATENTANMELDUNG

(11) **EP 0 714 906 A1**
(43) Veröffentlichungstag der Anmeldung: **05.06.1996**
(21) Anmeldenummer: 95116018.3
(22) Anmeldetag: 11.10.1995
(51) Int. Cl.: C07H 15/04, B01D 1/26

(54) **Verfahren zum destillativen Abtrennen von Fettalkoholen aus Alkylpolyglycosid-Lösungen**

(30) Priorität: 03.12.1994 DE 4443087
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein Verfahren zur destillativen Abtrennung von Fettalkoholen mit 8 bis 30 Kohlenstoffatomen aus fettalkoholischen Alkylpolyglycosid-Lösungen. Die Destillation erfolgt in mehreren Stufen, wobei ein oder mehr Fallfilmverdampfer und ein nachgeschalteter Kurzwegverdampfer verwendet werden.

## Beschreibung

Die Erfindung betrifft ein Verfahren zur destillativen Abtrennung von Fettalkoholen mit Kettenlängen von 8 bis 30, inbesondere von 8 bis 18 Kohlenstoffatomen, aus fettalkoholischen Alkylpolyglycosid-Lösungen.

Die Destillation erfolgt dabei in mehreren Stufen, wobei ein oder mehr Fallfilmverdampfer mit einem nachgeschalteten Kurzwegverdampfer kombiniert werden.

Alkylpolyglycoside sind ungiftige und leicht abbaubare oberflächenaktive Stoffe, die ganz oder teilweise aus nachwachsenden Rohstoffen hergestellt werden und die als Wasch- und Reinigungsmittel und als Emulgatoren und Dispergatoren verwendet werden. Die Alkylpolyglycoside können einstufig oder zweistufig durch Glycosidierung und Umglycosidierung hergestellt werden. Sie enthalten Alkylgruppen mit 8 bis 30, insbesondere mit 8 bis 18 Kohlenstoffatomen, wobei Alkylgruppen mit 12 bis 16 Kohlenstoffatomen besonders bevorzugt vorhanden sind. Der mittlere Polymerisationsgrad liegt bei 1,05 bis 1,5, vorzugsweise bei 1,2 bis 1,5 und ganz besonders bevorzugt bei 1,2 bis 1,4. Als einsetzbare Kohlenhydrate kommen Monosaccharide, wie Pentosen und Hexosen, Disaccharide, wie Saccharose und Maltose, und Polysaccharide, wie Stärke, in Betracht. Ein einstufiges Herstellungsverfahren wird u. a. in der Patentanmeldung P 41 01 252.6 beschrieben.
Ein zweistufiges Verfahren wird in EP-A-0 306 652 angegeben, wobei zunächst durch Glycosidierung mit n-Butanol ein n-Butylglycosid und daraus durch Umglycosidierung mit einem langkettigen Alkohol das gewünschte langkettige Alkylglycosid hergestellt wird.
Die Reaktion zwischen o. g. Kohlenhydraten und Fettalkoholen wird in der Regel mit einem großen Überschuß an Alkohol durchgeführt. Da dieser Alkoholüberschuß die anwendungstechnischen Eigenschaften der Alkylpolyglycoside stört, wird er nach Abschluß der Reaktion abgetrennt.
Da sich die Alkylpolyglycoside bzw. die nicht umgesetzten Kohlenhydratreste bei Temperaturen von über 150 °C unter Dunkelfärbung zersetzen, muß die destillative Abtrennung der Alkohole im Feinvakuum durchgeführt werden.
In DE 41 29 587 wird ein Verfahren zur Abtrennung der Fettalkohole mit Hilfe eines Dünnschichtverdampfers unter Einstellung einer Reynolds-Zahl von 30 bis 18 000 angegeben.
In EP 0 092 876 werden die Fettalkohole aus fettalkoholischen Alkylpolyglycosid-Lösungen ebenfalls mit Hilfe eines Dünnschichtverdampfers destilliert. Dabei soll die Reynolds-Zahl >20 000 betragen.
In DE 39 32 172 wird ein Verfahren angegeben, bei dem die nicht umgesetzten Alkohole in zwei Stufen abgetrennt werden, wobei in der ersten Stufe ein Fallfilmverdampfer und in der zweiten Stufe ein Dünnschichtverdampfer eingesetzt wird. Alle oben genannten Verfahren haben allerdings den Nachteil, daß das maximal erreichbare Vakuum im Dünnschichtverdampfer begrenzt ist (ca. 1 mbar), und daher eine für die temperaturempfindlichen Alkylpolyglycoside hohe Sumpftemperatur im Dünnschichtverdampfer eingestellt werden muß. Dies führt zu unerwünschten Produktverfärbungen, die in einem weiteren Aufarbeitungsschritt entfernt werden müssen.

Es bestand daher die Aufgabe, ein destillatives Aufarbeitungsverfahren für die fettalkoholischen Alkylpolyglycosidlösungen zu finden, das die thermische Belastung und die damit verbundene Dunkelfärbung minimiert.

Die Lösung dieser Aufgabe besteht in einem Verfahren, bei dem ein oder mehr Fallfilmverdampfer mit einem nachgeschalteten Kurzwegverdampfer kombiniert werden.

Gegenstand der Erfindung ist daher ein Verfahren zur destillativen Abtrennung von Fettalkoholen aus fettalkoholischen Alkylpolyglycosid-Lösungen, dadurch gekennzeichnet, daß ein oder mehr Fallfilmverdampfer und ein nachgeschalteter Kurzwegverdampfer verwendet werden.

Der bzw. die Fallfilmverdampfer werden mit einer Heizmitteltemperatur von 120 bis 160 °C, vorzugsweise 130 bis 150 °C, einem Betriebsdruck von 2 bis 20 mbar, vorzugsweise 3 bis 10 mbar, und einer Sumpftemperatur zwischen 100 und 140 °C, vorzugsweise 110 bis 130 °C, betrieben.
Die Anzahl der Fallfilmverdampfer beträgt vorzugsweise ein oder zwei.

Beim nachgeschalteten Kurzwegverdampfer beträgt die Heizmitteltemperatur 160 - 200 °C, der Betriebsdruck 0,1 - 1 mbar und die Sumpftemperatur 140 - 180 °C. Durch das im Kurzwegverdampfer erreichbare gute Vakuum kann die Sumpftemperatur - und damit die thermische Produktbelastung - deutlich reduziert werden, ohne daß mit einem Anstieg des Restfettalkohol-Gehaltes gerechnet werden muß.
Auf diese Weise sind deutlich hellere Produkte zu erhalten.

## Patentansprüche

1. Verfahren zur destillativen Abtrennung von Fettalkoholen aus fettalkoholischen Alkylpolyglycosid-Lösungen, dadurch gekennzeichnet, daß ein oder mehr Fallfilmverdampfer und ein nachgeschalteter Kurzwegverdampfer verwendet werden.

2. Verfahren nach Anspruch 1, dadurch gekennzeichnet, daß zwei Fallfilmverdampfer und ein nachgeschalteter Kurzwegverdampfer verwendet werden.

3. Verfahren nach Anspruch 1, dadurch gekennzeichnet, daß ein Fallfilmverdampfer und ein nachgeschalteter Kurzwegverdampfer verwendet werden.

4. Verfahren nach Anspruch 1, dadurch gekennzeichnet, daß die Fallfilmverdampfer mit einer Heizmitteltemperatur von 120 bis 160 °C, vorzugsweise 130 bis 150 °C, betrieben werden.

5. Verfahren nach Anspruch 1, dadurch gekennzeichnet, daß die Fallfilmverdampfer bei einem Betriebsdruck von 2 bis 20 mbar, vorzugsweise 3 bis 10 mbar, betrieben werden.

6. Verfahren nach Anspruch 1, dadurch gekennzeichnet, daß die Fallfilmverdampfer bei einer Sumpftemperatur von 100 bis 140 °C betrieben werden.

7. Verfahren nach Anspruch 1, dadurch gekennzeichnet, daß die Fallfilmverdampfer bei einer Sumpftemperatur von 110 bis 130 °C betrieben werden.

8. Verfahren nach den Ansprüchen 1 bis 7, dadurch gekennzeichnet, daß Fettalkohole mit 8 bis 30 Kohlenstoffatomen abgetrennt werden.

9. Verfahren nach Ansprüchen 1 bis 7, dadurch gekennzeichnet, daß Fettalkohole mit 8 bis 18 Kohlenstoffatomen abgetrennt werden.
